# NEUE EUROPÄISCHE PATENTSCHRIFT

(11) **EP 0 277 095 B2**
(45) Veröffentlichungstag und Bekanntmachung des Hinweises auf die Entscheidung über den Einspruch: **05.09.2001**
(45) Hinweis auf die Patenterteilung: 01.04.1992
(21) Anmeldenummer: 88810026.0
(22) Anmeldetag: 21.01.1988
(51) Int. Cl.: C07D 223/22, A61K 31/55

(54) **Verfahren zur Herstellung von N,N-(Dibenzohexatrienylen)Harnstoffen**
Method for preparing N,N-(dibenzohexatrienylene) ureas
Procédé pour la préparation de N,N-(dibenzohexatriénylène) urées

(30) Priorität: 27.01.1987 CH 27687
(43) Veröffentlichungstag der Anmeldung: 03.08.1988
(73) Patentinhaber: Novartis AG, 4056 Basel (CH)
(72) Erfinder: Acklin, Georg, Dr., CH-4144 Arlesheim (CH); Aufderhaar, Ernst, Dr., CH-4303 Kaiseraugst (CH); Kaupp, Günter, Dr., CH-4108 Witterswil (CH); Räz, Bernhard, CH-4056 Basel (CH); Vogel, Ulrich, Dr., CH-4312 Magden (CH)
(74) Vertreter: Zumstein, Fritz, Dr.

(56) Entgegenhaltungen:
- DE-C- 1 219 912
- CHEMISTRY AND INDUSTRY, 07 August 1965; Seiten 1428-1429
- Organic Syntheses, vol. 4, 1967, p. 49-51
- Advances in Heterocyclic Chemistry, vo. 9, Academic Press New York San Francisco Londom 1968, p. 329-332
- Advances in Heteroccyclic Chemistry, vol. 17, Academic Press New York San Francisco London, 1974, p. 257-266
- Beyer, Walter: Lehrbuch der Organischen Chemie, 20. Auflage, S. Hirzel Verlag Stuttgart, 1984, S. 332
- Acta Cryst., Vol. B32, 1976, S. 5-10
- Acta Cryst., Vol. B36, 1980, S. 2683-2688 und Zeitschrift für Kristallographie, Bd. 127, 1968, S. 456-459
- A. Kleemann und J. Engel, Pharmazeutische Wirkstoffe, 2. Auflage 1982, S. 144, 145
- Houben-Weyl, Methoden der Organische Chemie, Band E4, 1983, Seiten 362-364
- Organic Syntheses, 1943, Collective Volume 2, S. 79 und 80.

## Beschreibung

Die Erfindung betrifft ein Verfahren zur Herstellung von 5H-Dibenz[b,f]azepin-5-carboxamid dadurch gekennzeichnet, dass man 5H Dibenz[b,f]azepin
(i) in einem organischen Lösungsmittel oder Lösungsmittelgemisch und
(ii) in Gegenwart eines sauren Mittels ausgewählt aus einer Mineralsäure, einer organischen Sulfonsäure, einer aliphatischen Carbonsäure, einer 2-Mono-, einer 2,2-Di-halogen-C₂-C₇-alkansäure und Trichloressigsäure
(iii) mit Cyansäure umsetzt, welche durch
   (a) Pyrolyse aus Cyanursäure oder
   (b) Freisetzung aus Natriumcyanat mit einer Säure hergestellt wird, wobei das saure Mittel im Falle der in-situ-Freisetzung von Cyansäure aus Natriumcyanat in geringem Überschuss der für die Freisetzung des Cyansaüre erforderlichen Säuremenge verwendet wird.

5H-Dibenz[b,f]azepin-5-carboxamid, unter dem Freinamen Carbamazepin als Arzneimittelwirkstoff bekannt, wird gemäss US-PS 2,948,718 üblicherweise hergestellt durch Umsetzung von 5H-Dibenz[b,f]azepin mit Phosgen zu 5H-Dibenz[b,f]azepin-5-carbonsäurechlorid und Weiterumsetzung desselben mit Ammoniak. Einem neueren Verfahren gemäss der DE-A1 2307174 zufolge setzt man 5H-Dibenz[b,f]azepin mit einem Acylisocyanat um und unterwirft das gebildete 5H-Dibenz[b,f]azepin-5-(N-acyl)carboxamid der basischen Hydrolyse. Die bekannten Verfahren haben entschiedene Nachteile. So müssen stets zwei getrennte Reaktionsstufen durchgeführt werden, wobei für die erste Stufe des Verfahrens gemäss der US-PS die Verwendung einer äquimolaren Menge des hochtoxischen Phosgen unumgänglich ist.

Der Erfindung lag dementsprechend die bislang noch nicht gelöste Aufgabe zugrunde, ein Herstellungsverfahren zu entwickeln, das in einer Stufe direkt zu 5H-Dibenz[b,f]azepin-5-carboxamid führt.

Der erfindungsgemässe Lösungsvorschlag ist insofern überraschend, als bekannt ist, dass 5H-Dibenz[b,f]azepin mit Alkylisocyanaten nicht zu entsprechenden 5H-Dibenz[b,f]azepin-5-(N-alkyl)carboxamiden reagiert (DE-A1 2307174) und die Umsetzung von N,N-Diarylaminen mit Natriumcyanat und Trifluoressigsäure in Benzol nicht auf Benzimidazol und Carbazol übertragen werden konnte (Chem. and Ind. 1965, Seiten 1428-9).

Die erfindungsgemäss für die Einführung der 5-Carbamylgruppe verwendete Cyansäure wird hergestellt durch Pyrolyse von Cyanursäure oder durch Behandlung der Lösung und/oder Suspension von Natrium-cyanat, mit einer Säure. Cyansäure ist in freier Form nicht stabil. Sie geht eine Vielzahl von Polymerisations- und Autokondensationsreaktionen ein und addiert zudem leicht Wasser, Alkohole, Amine und dergleichen. Ihre Lösungen in geeigneten organischen Lösungsmitteln sind jedoch für den erfindungsgemässen Zweck hinreichend haltbar.

Die erfindungsgemässe Umsetzung erfolgt deshalb in organischer Lösung, d.h. in einem organischen Lösungsmittel oder Lösungsmittelgemisch, wobei die Cyansäure vorzugsweise in gasförmigem Zustand, vorteilhaft mit einem Inertgas, wie Stickstoff oder Argon verdünnt, in das Reaktionssystem eingeblasen oder durch Behandlung der Lösung und/oder Suspension von Natrium-cyanat, mit einer Säure in Freiheit gesetzt wird.

Als organische Lösungsmittel sind solche geeignet, die mit Cyansäure nicht oder nur so langsam reagieren, dass die erfindungsgemässe Reaktion nicht durch Bildung unerwünschter Zwischenprodukte beeinträchtigt wird. Geeignet sind beispielsweise aromatische bzw. araliphatische Kohlenwasserstoffe, wie Benzol oder Toluol, Halogenaliphaten, wie 1,2-Dichloräthan, aliphatische Carbonsäuren und ihre aliphatischen Ester, wie Niederalkancarbonsäuren, z.B. Essigsäure, oder Niederalkancarbonsäureniederalkylester, z.B. Essigsäureäthylester, ferner aliphatische Aether, wie Diäthyläther, Dioxan, Tetrahydrofuran und dergleichen, sowie Gemische derselben. Da Cyansäure mit Wasser, Alkoholen, Aminen und dergleichen unerwünschte Nebenreaktionen eingeht, wird die erfindungsgemässe Umsetzung vorteilhaft unter weitgehend aprotischen Bedingungen, d.h. in weitgehend wasser-, alkohol- und aminfreier organischer Lösung und unter Wasserdampfausschluss, durchgeführt. Bei der Aufarbeitung des Reaktionsgemisches und der Isolierung des gebildeten Additionsproduktes sind diese Vorsichtsmassnahmen jedoch völlig entbehrlich.

Für die erfindungsgemässe Umsetzung wird eine der eingesetzten Menge 5H-Dibenz[b,f]azepin mindestens äquimolare Cyansäuremenge benötigt Um einen besseren Umsatz zu erzielen, verwendet man jedoch vorteilhaft die 1,05-molare bis 2,5-molare, vorzugsweise 1,25-molare bis 2,25-molare, beispielsweise 1,3-molare bis doppeltmolare Menge, Cyansäure, d.h. einen 5%igen bis 150%igen, vorzugsweise 25%igen bis 125%igen, beispielsweise 30%igen bis 100%igen, Cyansäureüberschuss.

Für die Freisetzung von Cyansäure aus Natriumcyanat, welche eine besonders bevorzugte Ausführungsform der Erfindung darstellt, geeignet sind generell sämtliche Protonensäure, deren Säurestärke ausreicht, Cyansäure aus Natriumcyanat zu vedrängen. Geeignet sind beispielsweise Mineralsäuren, z.B. Chlorwasserstoffsäure oder Schwefelsäure, organische Sulfonsäuren, wie C₁-C₇-Alkan- oder gegebenenfalls halogen- oder C₁-C₄-alkylsubstituierte Benzolsulfonsäuren, z.B. Methan-, Aethan-, Benzol-, p-Toluol- oder p-Brombenzolsulfonsäure, oder aliphatische Carbonsäuren, deren Säurestärke in dem verwendeten Lösungsmittel mindestens derjenigen von Ameisensäure praktisch entspricht, wie 2-Mono-, 2,2-Di-halogen-C₂-C₇-alkansäuren, und Trichloressigsäure.

Die Reaktion der 5H-Dibenz[b,f]azepin-Komponente mit Cyansäure verläuft spontan und leicht exotherm. Die Reaktionsparameter sind nicht kritisch. Die Reaktion kann beispielsweise im Temperaturbereich von 0°C bis 120°C und homogen oder vorzugsweise heterogen durchgeführt werden. Gleichwohl werden Umsatz und Umsatzgeschwindigkeit durch gelindes Erwärmen und/oder Gegenwart eines sauren Mittels beschleunigt Die Umsetzung wird deshalb in Gegenwart eines sauren Mittels und vorzugsweise im Temperaturbereich von 20°C bis 120°C, vorzugsweise 100°C durchgeführt. Da das saure Mittel an der Reaktion nur katalytisch teilnimmt, genügen im Prinzip katalytische Säuremengen. Im allgemeinen sind 0,01 bis 0,15, beispielsweise 0,04 bis 0,05, Aequivalente saures Mittel je Mol 5H-Dibenz[b,f]azepin völlig ausreichend. Lediglich bei Verwendung mehrbasiger Säuren deutlich unterschiedlicher Aciditätsstufen ist bei heterogener Reaktionsführung zu beachten, dass saure Salze ausfallen können, wodurch ein Teil der eingesetzten Säure blockiert wird. Bei Verwendung von Schwefelsäure beispielsweise benötigt man deshalb je Mol 5H-Dibenz[b,f]azepin bis zu 1,5, beispielsweise 1,05 bis 1,4 Moläquivalente, entsprechend 0,525 bis 0,7 Mol, d.h. einen 5%igen bis 40%igen Ueberschuss, derselben, wenn die Variante, bei der man die Cyansäure aus Natriumcyanat freisetzt, benutzt und die Reaktion heterogen geführt wird. Selbstverständlich kann das saure katalytische Mittel auch in Form eines von 5H-Dibenz[b,f]azepin abgeleiteten Ammoniumsalzes vorliegen oder zugefügt werden.

Als saures Mittel kommen beispielsweise die vorstehend für die Freisetzung von Cyansäure als geeignet angegebenen Protonensäuren, ferner aliphatische Carbonsäuren, wie C₁-C₇-Alkansäuren, z.B. Essigsäure, insbesondere, wenn diese auch als Lösungsmittel dienen, in Betracht. Benutzt man die Variante, bei der die Cyansäure aus Natriumcyanat in situ freigesetzt wird, verwendet man vorteilhaft im allgemeinen einen geringen, d.h. 0,5%igen bis 10%igen, beispielsweise 1%igen bis 5%igen, bei Verwendung z.B. von Schwefelsäure jedoch aus den genannten Gründen einen 5%igen bis 40%igen, beispielsweise 32%igen, Ueberschuss der für die Freisetzung der Cyansäure verwendeten Säure.

In einer bevorzugten Ausführungsform fügt man zu einer Suspension von 5H-Dibenz[a,f]azepin und der 1,75- bis 2,25-molaren, beispielsweise etwa doppeltmolaren, Menge Natriumcyanat in Toluol bei 20°C bis 30°C, beispielsweise bei 20°C bis 25°C, pro Mol Natriumcyanat 1,005 bis 1,05, beispielsweise 1,02 Mol, d.h. einen 0,5%igen bis 5%igen, beispielsweise 2%igen Ueberschuss, Trichloressigsäure hinzu und erwärmt auf 40°C bis 80°C, beispielsweise auf 50°C bis 65°C, oder versetzt eine Suspension von 5H-Dibenz[b,f]azepin in Essigsäure mit 1,05 bis 1,40 Moläquivalenten, entsprechend 0,525 bis 0,7 Mol, d.h. mit einem 5%igen bis 40%igen Ueberschuss, Schwefelsäure und fügt dann die der eingesetzten Menge 5H-Dibenz-[b,f]azepin mindestens äquimolare Menge, beispielsweise pro Mol 5H-Di-benz[b,f]azepin 1,25 bis 1,75, z.B. 1,6 Mol, Natriumcyanat hinzu, wobei man bei 10°C bis 120°C arbeitet, oder leitet in eine Suspension von Natriumcyanat in Essigsäureäthylester die 1,02- bis 1,10-, beispielsweise 1,05-, d.h. 1,04- bis 1,06-molare Menge, d.h. einen 2%igen bis 10%igen, beispielsweise 5%igen, d.h. 4%igen bis 6%igen, Ueberschuss, Chlorwasserstoff ein und dann eine der eingesetzten Natriumcyanatmenge höchstens äquimolekulare Menge, beispielsweise einen 5%igen bis 50%igen molaren Unterschuss, an 5H-Dibenz[b,f]azepin beispielsweise pro Mol Natriumcyanat 0,6 bis 0,9, z.B. 0,75 Mol, 5H-Dibenz[b,f]azepin hinzu, wobei man bei 0°C bis 80°C arbeitet, beispielsweise nach Zugabe der Aminkomponente auf 40°C bis 70°C erwärmt.

In einer anderen bevorzugten Ausführungsform leitet man in eine Suspension von 5H-Dibenz[b,f]azepin in Essigsäure die 1,25-molare bis 1,75-molare, vorzugsweise 1,4-molare bis 1,6-molare Menge, d.h. einen 25%igen bis 75%igen, vorzugsweise 40%igen bis 60%igen, Ueberschuss, Cyansäure ein und erwärmt erforderlichenfalls auf 25°C bis 50°C oder leitet in eine Suspension von 5H-Dibenz[b,f]azepin in Toluol, Xylol, 1,2-Dichloräthan oder Essigsäureäthylester zunächst die 0,01-molare bis 0,15-molare, beispielsweise 0,01-molare bis 0,12-molare Menge, d.h. 1 bis 15 Mol-%, beispielsweise 1 bis 12 Mol-%, Chlorwasserstoff und anschliessend die 1,25-molare bis 1,75-molare, vorzugsweise 1,4-molare bis 1,6-molare Menge, d.h. einen beispielsweise 25%igen bis 75%igen, vorzugsweise 40%igen bis 60%igen Ueberschuss, Cyansäure ein und erwärmt erforderlichenfalls auf 50°C bis 125°C, beispielswelse auf 75°C bis 100°C. In einer Abwandlung dieser Variante leitet man in eine Suspension eines Gemisches des 5H-Dibenz[b,f]azepin und seines Hydrochlorides, beispielsweise von 0,8 bis 0,96, vorzugsweise 0,85 bis 0,95, Molteilen 5H-Dibenz-[b,f]azepin und 0,04 bis 0,2, vorzugsweise 0,05 bis 0,15, Molteilen 5H-Dibenz[b,f]azepin (Molteilsumme=1), die 1,25-molare bis 1,75-molare, vorzugsweise 1,4-molare bis 1,6-molare Menge, d.h. einen beispielsweise 25%igen bis 75%igen, vorzugsweise 40%igen bis 60%igen, Ueberschuss Cyansäure ein und erwärmt erforderlichenfalls auf 60°C bis 100°C.

Die Erfindung ist in den nachstehenden Ausführungsbeispielen näher beschrieben. Temperaturen sind in Celsiusgraden angegeben.

### Beispiel 1:

25 g 5H-Dibenz[b,f]azepin werden in 180 ml Essigsäure suspendiert und mit 14 g 96%iger Schwefelsäure langsam versetzt. Bei 30°C werden unter gutem Rühren 13,5 g Natriumcyanat portionsweise zugesetzt

Man rührt 3 Stunden bei 30°C, filtriert das Produkt ab und wäscht mit Essigsäure und anschliessend Wasser.

Nach Trocknen bei 80°C im Vakuum erhält man 29,5 g 5H-Dibenz[b,f]azepin-5-carboxamid.

### Beispiel 2:

Man suspendiert 68 g Natriumcyanat in 1000 ml Essigsäureäthylester und leitet unter Rühren bei Raumtemperatur 40 g Chlorwasserstoff gasförmig ein. Nach 4 Stunden wird das gebildete Natriumchlorid abfiltriert und das klare Filtrat mit 155 g 5H-Dibenz[b,f]azepin versetzt Man hält das Reaktionsgemisch für 4-5 Stunden auf 50°C, kühlt auf 0°C und filtriert das Produkt ab. Nach Waschen mit wenig Essigsäureäthylester und Trocknen bei 80°C im Vakuum erhält man 177 g 5H-Dibenz[b,f]azepin-5-carboxamid.

### Beispiel 3:

17.4 g 5H-Dibenz[b,f]azepin und 2.3 g 5H-Dibenz[b,f]azepin-hydrochlorid werden in 250 ml Toluol suspendiert. Man erwärmt auf 80° und leitet im Lauf von 1 1/2 Stunden 6.5 g monomere Cyansäure im Stickstoffstrom ein und heizt anschliessend noch 1/2 Stunde auf 100°C.

Nach Abkühlen auf 5° wird abfiltriert, viermal mit kaltem Toluol gewaschen und bei 60° im Vakuum getrocknet. Man erhält 18.5 g 5H-Dibenz[b,f]azepin-5-carboxamid.

### Beispiel 4:

17.4 g 5H-Dibenz[b,f]azepin und 2.3 g 5H-Dibenz[b,f]azepin-hydrochlorid werden in 250 ml Xylol (Isomerengemisch) suspendiert Bei 20° leitet man 6.5 g monomere Cyansäure im Stickstoffstrom ein und lässt 4 Stunden bei 30° nachreagieren.

Anschliessend wird auf 0° abgekühlt, filtriert und mit Xylol gewaschen. Nach Trocknen bei 80° im Vakuum erhält man 22.1 g 5H-Dibenz[b,f]azepin-5-carboxamid.

### Beispiel 5:

19.3 5H-Dibenz[b,f]azepin werden in 200 ml 1,2-Dichloräthan suspendiert. Bei 25° leitet man zuerst 4.5 g Chlorwasserstoff und dann 6.5 g Cyansäure gasförmig (im Stickstoffstrom) ein. Das Einleiten erfolgt während 5 Stunden in mehreren Portionen. Man lässt 1 Stunde nachreagieren, filtriert ab und wäscht mit 1,2-Dichloräthan, danach mit Wasser.

Nach Trocknen bei 60° im Vakuum erhält man 16.0 g 5H-Dibenz[b,f]azepin-5-carboxamid.

Ein gleichartig durchgeführter Ansatz wurde nach beendeter Reaktion eingedampft, der Rückstand mit Toluol kalt digeriert und abfiltriert. Nach Waschen mit Toluol und Wasser und Trocknen im Vakuum bie 60° wurden 22.5 g 5H-Dibenz[b,f]azepin-5-carboxamid erhalten.

### Beispiel 6

29.0 g 5H-Dibenz[b,f]azepin werden in 150 ml Essigsäureäthylester bei 20° suspendiert Man leitet zuerst 0.6 g Chlorwasserstoff und dann 9.7 g Cyansäure gasförmig (im Stickstoffstrom) ein.

Nach 15-stündigem Rühren bei 20° wird abfiltriert, mit Essigsäureäthylester gewaschen und anschliessend bei 60° im Vakuum getrocknet. Man erhält 32.0 g 5H-Dibenz[b,f]azepin-5-carboxamid.

Ein anologer Versuch bei 50° Reaktionstemperatur lieferte 29.4 g 5H-Di-benz[b,f]azepin-5-carboxamid.

### Beispiel 7

19.3 g 5H-Dibenz[b,f]azepin werden in 200 ml Essigsäureäthylester suspendiert und mit 1,0 ml Schwefelsäure (98%ig) versetzt.

Bei 25° werden 6.5 g monomere Cyansäure (im Stickstoffstrom) eingeleitet. Man lässt über Nacht stehen, dampft dann im Vakuum zur Trockne ein und nimmt den Rückstand mit Toluol auf. Nach Filtration, Waschen mit Toluol und Wasser und Trocknen bei 80° im Vakuum erhält man 19.7 g 5H-Di-benz[b,f]azepin-5-carboxamid.

### Beispiel 8

19.3 g 5H-Dibenz[b,f]azepin werden mit 100 ml Essigsäure auf 45° erwärmt. Im Lauf von 1 1/2 Stunden leitet man 6.5 g monomere Cyansäure (im Stickstoffstrom) ein und lässt 12 Stunden bei 40° nachreagieren. Nach Abkühlen auf 15° wird filtriert, mit Essigsäure kalt gewaschen und im Vakuum bei 60° getrocknet.

Das erhaltene Rohprodukt wird aus Methanol/Wasser (7:3) umkristallisiert und liefert 19.1 g 5H-Dibenz[b,f]azepin-5-carboxamid.

### Beispiel 9:

29.0 g 5H-Dibenz[b,f]azepin werden in 150 ml Essigsäure auf 45° erwärmt. Man leitet im Lauf von 1 1/2 Stunden 9.7 g monomere Cyansäure (im Stickstoffstrom) ein und lässt 2 Stunden bei 40° und 12 Stunden bei 20° nachreagieren.

Nach Zusatz von 15 ml Wasser wird auf 0° abgekühlt und nach 1 Stunde abfiltriert. Man wäscht mit zweimal 15 ml Essigsäure und Wasser und erhält ein Rohprodukt, das nach Umkristallisieren aus Methanoll/Wasser (7:3) 29.1 g 5H-Dibenz[b,f]azepin-5-carboxamid liefert.

## Patentansprüche

1. Verfahren zur Herstellung von 5H-Dibenz[b,f]azepin-5-carboxamid **dadurch gekennzeichnet**, dass man 5H-Dibenz[b,f]azepin
(i) in einem organischen Lösungsmittel oder Lösungsmittelgemisch und
(ii) in Gegenwart eines sauren Mittels ausgewählt aus einer Mineralsäure, einer organischen Sulfonsäure, einer aliphatischen Carbonsäure, einer 2-Mono-, einer 2,2-Di-halogen-C₂-C₇-alkansäure und Trichloressigsäure
(iii) mit Cyansäure umsetzt, welche durch
(a) Pyrolyse aus Cyanursäure oder
(b) Freisetzung aus Natriumcyanat mit eine Säure hergestellt wird, wobei das saure Mittel im Falle der in-situ-Freisetzung von Cyansäure aus Natriumcyanat in geringem Überschuss der für die Freisetzung des Cyansäure erforderlichen Säuremenge verwendet wird.

2. Verfahren gemäss Anspruch 1, **dadurch gekennzeichnet**, dass man als organisches Lösungsmittel eine aliphatische Carbonsäure oder einen aliphatischen Ester einer solchen, einen aromatischen bzw. araliphatischen Kohlenwasserstoff, einen Halogenaliphaten oder einen aliphatischen Aether verwendet.

3. Verfahren gemäss Anspruch 1 oder 2, **dadurch gekennzeichnet**, dass man als organisches Lösungsmittel Toluc Xylol, 1,2-Dichloräthan, Essigsäure oder Essigsäureäthylester verwendet.

4. Verfahren gemäss Anspruch 1 oder 2, **dadurch gekennzeichnet**, dass man Cyansäure durch Säurebehandlung der Lösung und/oder Suspension von Natriumcyanat in einem organischen Lösungsmittel in Freiheit setzt und ohne Isolierung einsetzt.

5. Verfahren gemäss Anspruch 3, **dadurch gekennzeichnet**, dass man Cyansäure durch Säurebehandlung der Lösung und/oder Suspension von Natriumcyanat in einem organischen Lösungsmittel in Freiheit setzt und ohne Isolierung einsetzt.

6. Verfahren gemäss einem der Ansprüche 1, 2 und 4, **dadurch gekennzeichnet**, dass man als organisches Lösungsmittel Essigsäure, Essigsäureäthylester oder Toluol verwendet.

7. Verfahren gemäss einem der Ansprüche 3 und 5, **dadurch gekennzeichnet**, dass man als saures Mittel eine Mineralsäure, eine organische Sulfonsäure, eine aliphatische Carbonsäure, eine 2-Mono-, oder eine 2,2-Di-halogen-C₂-C₇-alkansäure verwendet.

8. Verfahren gemäss einem der Ansprüche 1, 2, 4 und 6, **dadurch gekennzeichnet**, dass man als saures Mittel eine Mineralsäure, eine organische Sulfonsäure oder eine 2-Mono-, oder eine 2,2-Di-halogen-C₂-C₇-alkansäure verwendet.

9. Verfahren gemäss einem der Ansprüche 1, 2, 4 und 6, **dadurch gekennzeichnet**, dass man als saures Mittel eine Mineralsäure verwendet.

10. Verfahren gemäss einem der Ansprüche 3, 5, 7 und 8, **dadurch gekennzeichnet**, dass man als saures Mittel Trichloressigsäure, Chlorwasserstoffsäure oder Schwefelsäure verwendet.

11. Verfahren gemäss einem der Ansprüche 1, 2, 4, 6 und 9, **dadurch gekennzeichnet**, dass man als saures Mittel Chlorwasserstoffsäure oder Schwefelsäure verwendet.

12. Verfahren gemäss einem der Ansprüche 3, 5 und 10, **dadurch gekennzeichnet**, dass man als saures Mittel Trichloressigsäure verwendet.

13. Verfahren gemäss einem der Ansprüche 1, 2, 6 und 8, **dadurch gekennzeichnet**, dass man als saures Mittel und gleichzeitig als Lösungsmittel Essigsäure verwendet.

14. Verfahren gemäss einem der Ansprüche 3, 5, 7, 10 und 12, **dadurch gekennzeichnet**, dass man im Temperaturbereich von 0°C bis 120°C arbeitet.

15. Verfahren gemäss einem der Ansprüche 1, 2, 4, 6, 8, 9, 11 und 13, **dadurch gekennzeichnet**, dass man im Temperaturbereich von 0°C bis 120°C arbeitet.

16. Verfahren gemäss einem der Ansprüche 1 bis 6, 10, 12, 14 und 15, **dadurch gekennzeichnet**, dass man eine Suspension des 5H-Dibenz[b,t]azepins und der 1,75- bis 2,25-molaren Menge Natriumcyanat in Toluol bei 20°C bis 30°C mit einem 0,5%igen Ueberschuss an Trichloressigsäure versetzt und auf 40°C bis 80°C erwärmt.

17. Verfahren gemäss einem der Ansprüche 1 bis 6, 10, 12, 14 und 15 **dadurch gekennzeichnet**, dass man eine Suspension von 5H-Dibenz[b,f]azepin und der etwa doppeltmolaren Menge Natriumcyanat in Toluol bei 20°C bis 25° mit einem 2%igen Ueberschuss an Trichloressigsäure versetzt und dann auf 50°C bis 65°C erwärmt.

18. Verfahren gemäss einem der Ansprüche 1 bis 10, 14 und 15, **dadurch gekennzeichnet**, dass man in eine Suspension von Natriumcyanat in Essigsäureäthylester einen 2%igen bis 10%igen Ueberschuss Chlorwasserstoff einleitet, dann die auf die eingesetzte Natriumcyanatmenge bezogen 0,6- bis 0,9-molare Menge 5H-Dibenz[b,f]azepin hinzufügt und auf 40°C bis 70°C erwärmt.

19. Verfahren gemäss einem der Ansprüche 1, 2, 4, 6, 8, 11 und 15 **dadurch gekennzeichnet**, dass man bei 0°C bis +80°C in eine Suspension von Natriumcyanat in Essigsäureäthylester einen 4%- bis 6%igen Ueberschuss Chlorwasserstoff einleitet und dann eine der eingesetzten Natriumcyanatmenge höchstens äquimolare Menge 5H-Dibenz[b,f]azepin hinzufügt.

20. Verfahren gemäss einem der Ansprüche 1, 2, 4, 6, 8, 9, 11 und 14, **dadurch gekennzeichnet**, dass man bei +10°C bis +120°C eine Suspension von 5H-Dibenz[b,f]azepin in Essigsäure mit einem 5%igen bis 40%igem Ueberschuss an Schwefelsäure versetzt und dann eine der eingesetzten Menge an 5H-Dibenz[b,f]azepin mindestens äquimolare Menge Natriumcyanat hinzufügt.

21. Verfahren gemäss einem der Ansprüche 1 bis 11 und 14, **dadurch gekennzeichnet**, dass man eine Suspension des 5H-Dibenz[b,f]azepin in Essigsäure mit einem 5%igen bis 40%igem Ueberschuss an Schwefelsäure versetzt und dann pro Mol 5H-Dibenz[b,f]azepin 1,25 bis 1,75 Mol Natriumcyanat hinzufügt.

22. Verfahren gemäss einem der Ansprüche 1 bis 11 und 14, **dadurch gekennzeichnet**, dass man bei +10°C bis +120°C eine Suspension von 5H-Dibenz[b,f]azepin in Essigsäure mit einem 5%igen bis 40%igem Ueberschuss an Schwefelsäure versetzt und dann pro Mol 5H-Dibenz[b,f]azepin 1,6 Mol Natriumcyanat hinzufügt.

23. Verfahren gemäss einem der Ansprüche 1 bis 3, 6, 7, 13 und 14, **dadurch gekennzeichnet**, dass man in eine Suspension des von 5H-Dibenz[b,f]azepin in Essigsäure einen 25%igen bis 75%igen Ueberschuss an Cyansäure einleitet.

24. Verfahren gemäss einem der Ansprüche 1 bis 3, 6, 7, 8 bis 11 und 14, **dadurch gekennzeichnet**, dass man in eine Suspension des von 5H-Dibenz[b,f]azepin in Toluol, Xylol, 1,2-Dichloräthan oder Essigsäureäthylester zunächst 1 bis 15 Mol-% Chlorwasserstoff und dann einen 25%igen bis 75%igen Ueberschuss an Cyansäure einleitet.

25. Verfahren gemäss einem der Ansprüche 1 bis 3, 6, 7, 8 bis 11 und 14, **dadurch gekennzeichnet**, dass man in die Suspension eines Gemisches von 0,8 bis 0,96 Molteilen des 5H-Dibenz[b,f]azepin und 0,04 bis 0,2 Molteilen 5H-Dibenz[b,f]azepin-hydrochlorid (Molteilsumme=1) in einem araliphatischen Kohlenwasserstoff einen 25%igen bis 75%igen Ueberschuss an Cyansäure einleitet.

## Claims

1. Process for the production of 5H-dibenz[b,f]azepine-5-carboxamide, **characterised in that** 5H-dibenz[b,f]azepine is reacted
(i) in an organic solvent or solvent mixture and
(ii) in the presence of an acidic medium selected from a mineral acid, an organic sulphonic acid, an aliphatic carboxylic acid, a 2-mono-, a 2,2-di-halo-C₂-C₇-alkanoic acid and trichloroacetic acid
(iii) with cyanic acid, which is produced by
(a) pyrolysis from cyanuric acid or
(b) release from sodium cyanate with an acid, whereby in the case of the *in situ* release of cyanic acid from sodium cyanate, the acidic medium is used in slight excess of the amount of acid required for releasing cyanic acid.

2. Process according to claim 1, **characterised in that** an aliphatic carboxylic acid or an aliphatic ester thereof, an aromatic or araliphatic hydrocarbon, a haloaliphatic compound or an aliphatic ether, is used as the organic solvent.

3. Process according to claim 1 or 2, **characterised in that** toluene, xylene, 1,2-dichloroethane, acetic acid or ethyl acetate is used as the organic solvent.

4. Process according to claim 1 or 2, **characterised in that** cyanic acid is released with the aid of an acid by treating the solution and/or suspension of sodium cyanate in an organic solvent, and it is used without isolation.

5. Process according to claim 3, **characterised in that** cyanic acid is released with the aid of an acid by treating the solution and/or suspension of sodium cyanate in an organic solvent, and it is used without isolation.

6. Process according to one of claims 1, 2 and 4, **characterised in that** acetic acid, ethyl acetate or toluene is used as the organic solvent.

7. Process according to one of claims 3 and 5, **characterised in that** a mineral acid, an organic sulphonic acid, an aliphatic carboxylic acid, a 2-mono- or a 2,2-dihalo-C₂-C₇-alkanoic acid is used as the acidic medium.

8. Process according to one of claims 1, 2, 4 and 6, **characterised in that** a mineral acid, an organic sulphonic acid or a 2-mono- or a 2,2-dihalo-C₂-C₇-alkanoic acid is used as the acidic medium.

9. Process according to one of claims 1, 2, 4 and 6, **characterised in that** a mineral acid is used as the acidic medium.

10. Process according to one of claims 3, 5, 7 and 8, **characterised in that** trichloroacetic acid, hydrochloric acid or sulphuric acid is used as the acidic medium.

11. Process according to one of claims 1, 2, 4, 6 and 9, **characterised in that** hydrochloric acid or sulphuric acid is used as the acidic medium.

12. Process according to one of claims 3, 5 and 10, **characterised in that** trichloroacetic acid is used as the acidic medium.

13. Process according to one of claims 1, 2, 4, 6 and 8, **characterised in that** acetic acid is used as the acidic medium and simultaneously as the solvent.

14. Process according to one of claims 3, 5, 7, 10 and 12, **characterised in that** it is carried out in a temperature range of 0°C to 120°C.

15. Process according to one of claims 1, 2, 4, 6, 8, 9, 11 and 13, **characterised in that** it is carried out in a temperature range of 0°C to 120°C.

16. Process according to one of claims 1 to 6, 10, 12, 14 and 15, **characterised in that** a suspension of 5H-dibenz[b,f]azepine and a 1.75 to 2.25 molar amount of sodium cyanate in toluene is mixed with a 0.5% excess of trichloroacetic acid at 20°C to 30°C, and the whole is heated to 40°C to 80°C.

17. Process according to one of claims 1 to 6, 10, 12, 14 and 15, **characterised in that** a suspension of 5H-dibenz[b,f]azepine and approximately double the molar amount of sodium cyanate in toluene is mixed with a 2% excess of trichloroacetic acid at 20°C to 25°C, and the whole is then heated to 50°C to 65°C.

18. Process according to one of claims 1 to 10, 14 and 15, **characterised in that** a 2% to 10% excess of hydrogen chloride is introduced into a suspension of sodium cyanate in ethyl acetate, and then a 0.6 to 0.9 molar amount of 5H-dibenz[b,f]azepine, based on the amount of sodium cyanate used, is added and the whole is heated to 40°C to 70°C.

19. Process according to one of claims 1, 2, 4, 6, 8, 11 and 15, **characterised in that** a 4% to 6% excess of hydrogen chloride is introduced into a suspension of sodium cyanate in ethyl acetate at 0°C to +80°C, and then an amount of 5H-dibenz[b,f]azepine, that is at most equimolar to the amount of sodium cyanate used, is added.

20. Process according to one of claims 1, 2, 4, 6, 8, 9, 11 and 14, **characterised in that** a suspension of 5H-dibenz[b,f]azepine in acetic acid is mixed with a 5% to 40% excess of sulphuric acid at +10°C to +120°C, and then an amount of sodium cyanate, that is at least equimolar to the amount of 5H-dibenz[b,f]azepine used, is added.

21. Process according to one of claims 1 to 11 and 14, **characterised in that** a suspension of 5H-dibenz[b,f]azepine in acetic acid is mixed with a 5% to 40% excess of sulphuric acid, and then 1.25 to 1.75 mols of sodium cyanate per mol of 5H-dibenz[b,f]azepine are added.

22. Process according to one of claims 1 to 11 and 14, **characterised in that** a suspension of 5H-dibenz[b,f]azepine in acetic acid is mixed with a 5% to 40% excess of sulphuric acid at +10°C to +120°C, and then 1.6 mols of sodium cyanate per mol of 5H-dibenz[b,f]azepine are added.

23. Process according to one of claims 1 to 3, 6, 7, 13 and 14, **characterised in that** a 25% to 75% excess of cyanic acid is introduced into a suspension of 5H-dibenz[b,f]azepine in acetic acid.

24. Process according to one of claims 1 to 3, 6, 7, 8 to 11 and 14, **characterised in that** first of all 1 to 15 mol % of hydrogen chloride and then a 25% to 75% excess of cyanic acid are introduced into a suspension of 5H-dibenz[b,f]azepine in toluene, xylene, 1,2-dichloroethane or ethyl acetate.

25. Process according to one of claims 1 to 3, 6, 7, 8 to 11 and 14, **characterised in that** a 25% to 75% excess of cyanic acid is introduced into the suspension of a mixture of 0.8 to 0.96 molar proportions of 5H-dibenz[b,f]azepine and 0.04 to 0.2 molar proportions of 5H-dibenz[b,f]azepine hydrochloride (total molar proportions = 1) in an araliphatic hydrocarbon.

## Revendications

1. Un procédé de préparation de 5H-dibenzo[b,f]azépine-5-carboxamide **caractérisé en ce qu**'on fait réagir la 5H-dibenzo[b,f]azépine
(i) dans un solvant organique ou un mélange de solvants et
(ii) en présence d'un agent acide choisi parmi un acide minéral, un acide sulfonique organique, un acide carboxylique aliphatique, un acide 2-mono-, 2,2-dihalogéno-C₂-C₇alcanoïque et un acide trichloroacétique
(iii) avec l'acide cyanique, lequel est préparé
(a) par pyrolyse à partir d'acide cyanurique ou bien
(b) par libération à partir de cyanate de sodium avec un acide, l'agent acide dans le cas d'une libération in situ de l'acide cyanique à partir de cyanate de sodium étant utilisé en faible excès de la quantité d'acide nécessaire pour la libération de l'acide cyanique.

2. Un procédé selon la revendication 1, **caractérisé en ce qu**'on utilise comme solvant organique, un acide carboxylique aliphatique ou un de ses esters aliphatiques, un hydrocarbure aromatique ou araliphatique, un hydrocarbure aliphatique halogéné ou un éther aliphatique.

3. Un procédé selon la revendication 1 ou 2, **caractérisé en ce qu**'on utilise comme solvant organique, le toluène, le xylène, le 1,2-dichloroéthane, l'acide acétique ou l'acétate d'éthyle.

4. Un procédé selon la revendication 1 ou 2, **caractérisé en ce que** l'acide cyanique est libéré par traitement acide d'une solution et/ou d'une suspension de cyanate de sodium dans un solvant organique et est utilisé sans isolement.

5. Un procédé selon la revendication 3, **caractérisé en ce que** l'acide cyanique est libéré par traitement acide d'une solution et/ou d'une suspension de cyanate de sodium dans un solvant organique et est utilisé sans isolement.

6. Un procédé selon l'une quelconque des revendications 1, 2 et 4, **caractérisé en ce que**, comme solvant organique, on utilise, l'acide acétique, l'acétate d'éthyle ou le toluène.

7. Un procédé selon l'une quelconque des revendications 3 et 5, **caractérisé en ce que**, comme agent acide, on utilise un acide minéral, un acide sulfonique organique, un acide carboxylique aliphatique, un acide 2-mono- ou 2,2,-dihalogéno-C₂-C₇-alcananoïque.

8. Un procédé selon l'une quelconque des revendications 1, 2, 4 et 6, **caractérisé en ce que**, comme agent acide, on utilise un acide minéral, un acide sulfonique organique ou un acide 2-mono- ou 2,2,-dihalogéno-C₂-C₇-alcanoïque.

9. Un procédé selon l'une quelconque des revendications 1, 2, 4 et 6, **caractérisé en ce que**, comme agent acide, on utilise un acide minéral.

10. Un procédé selon l'une quelconque des revendications 3, 5, 7 et 8, **caractérisé en ce que**, comme agent acide, on utilise l'acide trichloroacétique, l'acide chlorhydrique ou l'acide sulfurique.

11. Un procédé selon l'une quelconque des revendications 1, 2, 4, 6 et 9, **caractérisé en ce que**, comme agent acide, on utilise l'acide chlorhydrique ou l'acide sulfurique.

12. Un procédé selon l'une quelconque des revendications 3, 5 et 10, **caractérisé en ce que**, comme agent acide, on utilise de l'acide trichloroacétique.

13. Un procédé selon l'une quelconque des revendications 1, 2, 6 et 8, **caractérisé en ce que**, comme agent acide et en même temps comme solvant, on utilise l'acide acétique.

14. Un procédé selon l'une quelconque des revendications 3, 5, 7, 10 et 12, **caractérisé en ce qu**'on opère dans un intervalle de température de 0°C à 120°C.

15. Un procédé selon l'une quelconque des revendications 1, 2, 4, 6, 8, 9, 11 et 13, **caractérisé en ce qu**'on opère dans un intervalle de température de 0°C à 120°C.

16. Un procédé selon l'une quelconque des revendications 1 à 6, 10, 12, 14 et 15, **caractérisé en ce qu**'à une suspension de 5H-dibenzo[b,f]azépine et d'une quantité 1,75 à 2,25 molaire de cyanate de sodium dans du toluène entre 20°C et 30°C, on ajoute un excès de 0,5% d'acide trichloroacétique et on chauffe à 40°C à 80°C.

17. Un procédé selon l'une quelconque des revendications 1 à 6, 10, 12, 14 et 15, **caractérisé en ce qu**'à une suspension de 5H-dibenzo[b,f]azépine et à la quantité environ 2 fois molaire de cyanate de sodium dans du toluène, entre 20°C et 25°C, on ajoute un excès de 2% d'acide trichloroacétique et on chauffe ensuite à 50°C à 65°C.

18. Un procédé selon l'une quelconque des revendications 1 à 10, 14 et 15, **caractérisé en ce que**, dans une suspension de cyanate de sodium dans de l'acétate d'éthyle, on introduit un excès de 2% à 10% d'acide chlorhydrique, on ajoute ensuite la quantité 0,6 à 0,9 molaire de 5H-dibenzo[b,f]azépine par rapport à la quantité de cyanate de sodium utilisée et on chauffe à 40°C à 70°C.

19. Un procédé selon l'une quelconque des revendications 1, 2, 4, 6, 8, 11 et 15, **caractérisé en ce qu**'entre 0°C et + 80°C, dans une suspension de cyanate de sodium dans de l'acétate d'éthyle, on introduit un excès de 4% à 6% d'acide chlorhydrique et on ajoute ensuite à la quantité de cyanate de sodium utilisée, la quantité au plus équimolaire de 5H-dibenzo[b,f,]-azépine.

20. Un procédé selon l'une quelconque des revendications 1, 2, 4, 6, 8, 9, 11 et 14, **caractérisé en ce qu**'entre +10°C et + 120°C, à une suspension de 5H-dibenzo-[b,f,]azépine dans de l'acide acétique, on ajoute un excès de 5% à 40% d'acide sulfurique et on ajoute ensuite une quantité de cyanate de sodium au moins équimolaire par rapport à la quantité de 5H-dibenzo[b,f]azépine utilisée.

21. Un procédé selon l'une quelconque des revendications 1 à 11 et 14, **caractérisé en ce qu**'à une suspension de 5H-dibenzo[b,f]azépine dans de l'acide acétique, on ajoute un excès de 5% à 40% d'acide sulfurique et on ajoute ensuite par mole de 5H-dibenzo[b,f]azépine, 1,25 à 1,75 mole de cyanate de sodium.

22. Un procédé selon l'une quelconque des revendications 1 à 11 et 14, caratérisé en ce qu'entre +10°C et +120°C, à une suspension de 5H-dibenzo[b,f,]-azépine dans de l'acide acétique, on ajoute un excès de 5% à 40% d'acide sulfurique et on ajoute ensuite par mole de 5H-dibenzo[b,f]azépine, 1,6 mole de cyanate de sodium.

23. Un procédé selon l'une quelconque des revendications 1 à 3, 6, 7, 13 et 14, **caractérisé en ce que**, dans une suspension de 5H-dibenzo[b,f,]azépine dans de l'acide acétique, on introduit un excès de 25% à 75% d'acide cyanique.

24. Un procédé selon l'une quelconque des revendications 1 à 3, 6, 7, 8 à 11 et 14, **caractérisé en ce que**, dans une suspension de 5H-dibenzo[b,f,]azépine dans du toluène, du xylène, du 1,2-dichloroéthane ou de l'acétate d'éthyle, on introduit d'abord 1 à 15 % molaire d'acide chlorhydrique et ensuite un excès de 25% à 75% d'acide cyanique.

25. Un procédé selon l'une quelconque des revendications 1 à 3, 6, 7, 8 à 11 et 14, **caractérisé en ce que**, dans une suspension d'un mélange de 0,8 à 0,96 partie molaire de 5H-dibenzo[b,f,]azépine et 0,04 à 0,2 partie molaire de chlorhydrate de 5H-dibenzo[b,f,]azépine (somme des parties molaires = 1) dans un hydrocarbure araliphatique, on introduit un excès de 25% à 75% d'acide cyanique.
